Europäisches Patentamt

**European Patent Office**

**Office européen des brevets**

(11) Publication number: **0 057 323**
A2

# EUROPEAN PATENT APPLICATION

(21) Application number: **81305980.5**

(22) Date of filing: **21.12.81**

(51) Int. Cl.³: **A 61 K 7/16**

(30) Priority: **24.12.80 US 220223**

(43) Date of publication of application: **11.08.82**
**Bulletin 82/32**

(84) Designated Contracting States: **AT BE CH DE FR GB IT LI LU NL SE**

(71) Applicant: **WARNER-LAMBERT COMPANY, 201 Tabor Road, Morris Plains New Jersey 07950 (US)**

(72) Inventor: **Dills, Steven Searight, 1100 Parsippany Boulevard Apt. 213, Parsippany New Jersey 07054 (US)**

(74) Representative: **Jones, Michael Raymond et al, Haseltine Lake & Co. 28 Southampton Buildings Chancery Lane, London WC2A 1AT (GB)**

(54) **Anticaries composition.**

(57) A composition useful for reducing the incidence and severity of human dental caries is disclosed. The composition comprises an aminated cariogen (such as glucosamine, N-acetylglucosamine or mannosamine) or a pharmaceutically acceptable salt thereof, in association with a pharmaceutically acceptable carrier. Such a composition inhibits the cariogen induced production of acid by human dental plaque bacteria.

EP 0 057 323 A2

-1-

ANTICARIES COMPOSITION

This invention relates to an anticaries composition for inhibiting the cariogen induced production of acid by human dental plaque bacteria, thereby reducing the incidence or severity of human dental caries.

It is believed that certain acidogenic bacteria, in particular Streptococcus mutans and several Lactobacillus species, e.g., L. acidophilus, are the predominant cariogenic microorganisms present in human dental plaque. These microorganisms when presented with metabolizable carbohydrates, such as glucose, fructose, lactose, maltose and sucrose, produce acid metabolic end products, e.g. acetic acid, formic acid and lactic acid. These acids are believed to be the major causative agents of human dental caries. It would therefore be beneficial to provide a means whereby the carbohydrate induced acid production by human dental plaque bacteria would be inhibited, thereby reducing the incidence and severity of human dental caries.

According to the present invention, there is provided a composition for reducing the incidence or severity of human dental caries, the composition comprising an aminated cariogen or a pharmaceutically acceptable salt thereof in association with a pharmaceutically acceptable carrier.

The present invention also provides, for use in reducing the incidence or severity of human dental caries, an aminated cariogen or a pharmaceutically acceptable salt thereof.

The term "cariogen" used herein means any carbohydrate that is metabolized by human dental plaque bacteria to cause human dental caries. Examples of such cariogens are glucose, fructose, maltose, lactose, sucrose and the like.

The term "aminated cariogen" used herein means any monosaccharide or disaccharide wherein at least one hydroxyl group has been replaced by an amine group. In addition, the amine group may be substituted by a $C_{1-6}$ alkyl group such as methyl or a $C_{1-6}$ acyl group such as acetyl. The aminated cariogens may, in addition, be alkylated, alkoxylated, halogenated, phosphorylated, sulfated and esterified. Deoxy and sulfur derivatives are also contemplated.

It is now recognized that the D-isomers of the aminated cariogens are active; however, at this time there is no reason to believe that certain L-isomers may not also be useful. Therefore, the term "aminated cariogen" is intended to include the D-isomers, the L-isomers and mixtures thereof.

The present invention involves the discovery that aminated cariogens will effectively limit the decline in pH which normally occurs when human dental plaque microorganisms are presented with a cariogen.

It is known that carbohydrate induced acid production by Streptococcus mutans (one of the primary acidogenic microorganisms present in human dental plaque) is inhibited by certain monosaccharide analogues, e.g. 2-deoxy-D-glucose (see C.F. Schachtele and W.S. Leung, J.Dent. Res., 54 433 (1975)). However, it has been proposed that the in vivo use of certain of these analogues is not practical since they interfere with mammalian metabolism (see K. R. Roberts and M.L. Hayes, Scand. J. Dent. Res., 88, 201 (1980)). Aminated derivatives of monosaccharides and disaccharides are not known to possess this same untoward effect. In fact, glucosamine and N-acetylglucosamine, both aminated derivatives of the monosaccharide glucose, are known pharmaceutical aids and are currently marketed in the United States combined with oxytetracycline, a broad spectrum antibiotic (Physician's Desk Reference 34ed., 1980, p. 1374).

For a better understanding of the invention, reference will now be made, by way of example, to the accompanying drawings, which are graphs showing the production of acid (measured on the ordinate as pH) relative to time (measured on the abscissa in minutes). In the drawings:

Figure I relates to the effect of D-glucosamine hydrochloride on the production of acid by Streptococcus mutans g 6715 from D-fructose, wherein:

line 1 relates to the use of water as a control,
line 2 relates to the use of 10 mM of fructose plus 10 mM of D-glucosamine hydrochloride,
line 3 relates to the use of 10 mM of fructose plus 5 mM of D-glucosamine hydrochloride,
line 4 relates to the use of 10 mM of fructose plus 2.5 mM of D-glucosamine hydrochloride,
line 5 relates to the use of 10 mM of fructose, and
line 6 relates to the use of 10 mM of D-glucosamine hydrochloride;

Figure II relates to the effect of N-acetyl-D-glucosamine on the production of acid by Streptococcus mutans g 6715 from fructose, wherein:

line 1 relates to the use of water as a control,
line 2 relates to the use of 10 mM of fructose plus 10 mM of N-acetyl-D-glucosamine,
line 3 relates to the use of 10 mM of fructose plus 5 mM of N-acetyl-D-glucosamine,
line 4 relates to the use of 10 mM of fructose plus 2.5 mM of N-acetyl-D-glucosamine,
line 5 relates to the use of 10 mM of fructose, and

0057323

line 6 relates to the use of 10 mM of N-acetyl-D-glucosamine;

Figure III relates to the effect of D-glucosamine hydrochloride on the production of acid by <u>Streptococcus mutans</u> g 6715 from sucrose, wherein:

line 1 relates to the use of water as a control,

line 2 relates to the use of 10 mM of sucrose plus 5 mM of D-glucosamine hydrochloride,

line 3 relates to the use of 10 mM of sucrose plus 1 mM of D-glucosamine hydrochloride, and

line 4 relates to the use of 10 mM of sucrose.

To obtain the results shown in the Figures, cell suspensions of <u>Streptococcus mutans</u> were prepared by harvesting log phase cell cultures, washing them with 1 mm potassium phosphate buffer, pH 7.0., then resuspending them to 100 mg cell dry weight per ml. Measurements of acid production following cariogen addition were made by monitoring hydrogen ion concentrations over time with a Radiometer RTS 822 Titration Recording System. Assay conditions were as follows: temperature, $37^{\circ}$C; constant stirring; two ml reaction volume; 1 mM potassium phosphate; and 0.1 to 10 mg cell dry weight. Cariogen and inhibitor

additions are specified with the experiment.   0057323

In figure I, acid production by <u>Streptococcus</u> <u>mutans</u> was monitored by pH measurements of the cell suspension medium for 20 minutes following addition of varying amounts of D-glucosamine and fructose. The addition of fructose alone caused a rapid pH decline to pH 4.5 within 4 minutes. The rate of decline then decreased, finally reaching a pH of 3.8 at 20 minutes. D-Glucosamine addition at 2.5 mM and higher concentrations almost totally eliminated the acid production from fructose, while no acid production was detected using D-glucosamine alone. Similar results were obtained when testing the effects of N-acetyl-D-glucosamine on acid production from fructose, figure II, and the effects of D-glucosamine on acid production from sucrose, figure III; both by <u>Streptococcus</u> <u>mutans</u>.

The mechanism whereby aminated cariogens inhibit acid production by human dental plaque bacteria is probably twofold:

1)   inhibition of cariogen uptake by plaque bacteria; and

2)   the buffering capacity possessed by the aminated cariogens.

Direct evidence for the inhibition of cariogen uptake by hexosamines, for example, is shown in Table I. These data show that various hexosamines including, D-glucosamine-HCl, D-mannosamine-HCl and N-acetyl-D-glucosamine significantly inhibit the initial uptake of radioactively labeled fructose by <u>Streptococcus</u> <u>mutans</u> cells. These inhibitory effects have not previously been reported.

Hexosamines have $pk_a$ values of approximately 7.8, and their buffering capacity is well known (R. Dawson, et al., Data for Biochemical Research, Clarendon Press, Oxford, England, 1969, p. 232).

TABLE I

0057323

Inhibition by Hexosamines of D-Fructose Initial
Uptake by Streptococcus mutans.*

| Inhibitor** | Uptake*** |
|---|---|
| None | 16.1 |
| D-Glucosamine HCl | 6.6 |
| D-Mannosamine HCl | 11.5 |
| D-Galactosamine HCl | 18.2 |
| N-Acetyl-D-Glucosamine | 6.9 |

\*     Cells were grown and prepared as described
in the text explanations for figures I-III.

\*\*     Inhibitors, final concentration 1.0 mM, were
added simultaneously with fructose.

\*\*\*     Data is given as nmols fructose taken up per
min per mg of cell protein, [cell protein
was determined by the method of O. H. Lowry,
et al., J. Biol. Chem., 193, 265 (1951)].

Salts of the aminated cariogens with pharmaceutically acceptable acids are considered equivalent to the corresponding free base forms for purposes of the invention. Examples of suitable pharmaceutically acceptable acids for preparing such salts are hydrochloric, sulfuric, acetic, benzoic, hydrofluoric, fumaric, citric, phosphoric, malic and the like.

Specific examples of the aminated cariogens contemplated by the invention are glucosamine, glucosamine hydrochloride, N-acetylglucosamine, N-methylglucosamine, mannosamine, galactosamine, $\alpha$-2-deoxy-2-amino-glucopyranosyl-$\beta$-fructofuranose and the like. Preferred are glucosamine, N-acetylglucosamine, glucosamine hydrochloride and mannosamine.

Mixtures of aminated derivatives of monosaccharides, mixtures of aminated derivatives of disaccharides as well as mixtures of aminated derivatives of mono and disaccharides may also be utilized as the aminated cariogen.

The pharmaceutically acceptable carriers contemplated by the invention are well known to those skilled in the art and are exemplified by any substantially non-toxic material or mixture of materials which can be utilized to place the aminated cariogen in contact with human dental plaque, and thereby in contact with human dental plaque bacteria which are exemplified by species such as Streptococcus mutans, Lactobacillus acidophilus and the like. This contact is intended to take place in the mouth. Thus, simply providing the aminated cariogen in solution or suspension for use as a mouth rinse is sufficient. The addition of colorants, detergents, flavors, sweeteners, stabilizers and other adjuvants to such a solution or suspension is optional. In addition, the aminated cariogen may be added to any standard preparation normally used in the mouth; such as mouth rinses, mouth washes, mouth deoderizers, gargles, toothpastes, tooth powders and the like. The aminated cariogen is also suited to be added to chewing gums, hard candies, pressed mints, ice cream, beverages, cakes, pies, jams, jellies and to any other cariogen containing food product.

The aminated cariogens are particularly suited to be added to cariogen containing food products which remain in the mouth for an extended period of time. Carbohydrate sweetened confections such as candies or chewing gums are intended to remain in the mouth for extended periods. These confections may derive all or a portion of their sweetness from metabolizable carbohydrates such as fructose and/or sucrose which will lead to prolonged acidic conditions. This is due to the organic acid metabolic end products which are produced by the human dental plaque bacteria. The prolonged acidic conditions of course, will lead to tooth demineralization. The presence of an aminated cariogen with such confections in the mouth will effectively reduce acid production, thereby limiting the incidence and severity of human dental caries caused by the carbohydrates present in such confections.

The aminated cariogens may be present in a wide range of concentrations which would not be deleterious to a human receiving said aminated derivative. Thus, the aminated cariogen may be present in concentrations ranging from about 1 mmolar to about 500 mmolar. The preferred concentration range is from about 10 mmolar to about 100 mmolar.

The following are examples of confections and oral care product formulations which may be used to place the aminated cariogen in contact with human dental plaque. It will be understood that these formulations may be produced using standard methods, standard machinery and standard manufacturing techniques.

0057323

## Dentifrice

| | | |
|---|---|---|
| Dicalcium phosphate | 45 | % |
| Glycerine | 8 | % |
| Detergent | 10 | % |
| Whitener | 1 | % |
| Flavor including sweetener | 1 | % |
| Thickener and emulsifiers | 17 | % |
| Glucosamine hydrochloride | 5 | % |
| Water | q.s. | |
| | 100 | % |

## Oral Rinse

| | | |
|---|---|---|
| Flavor | 1 | % |
| Alcohol | 20 | % |
| Sweeteners | 1 | % |
| Thickener | .6 | % |
| Coloring Agent | .01 | % |
| Glycerine | 7 | % |
| Glucosamine hydrochloride | 5 | % |
| Water | q.s. | |
| | 100 | % |

## Chewing Gum

| | | |
|---|---|---|
| Sucrose | 60 | % |
| Corn Syrup | 10 | % |
| Flavor | 1 | % |
| Gum Base | 19 | % |
| Glucosamine hydrochloride | 10 | % |
| | 100 | % |

## Pressed Mint

| | | |
|---|---|---|
| Sucrose | 90 | % |
| Corn Syrup | 3 | % |
| Magnesium Stearate | 0.5 | % |
| Flavor | 0.5 | % |
| Glucosamine Hydrochloride | 6 | % |
| | 100 | % |

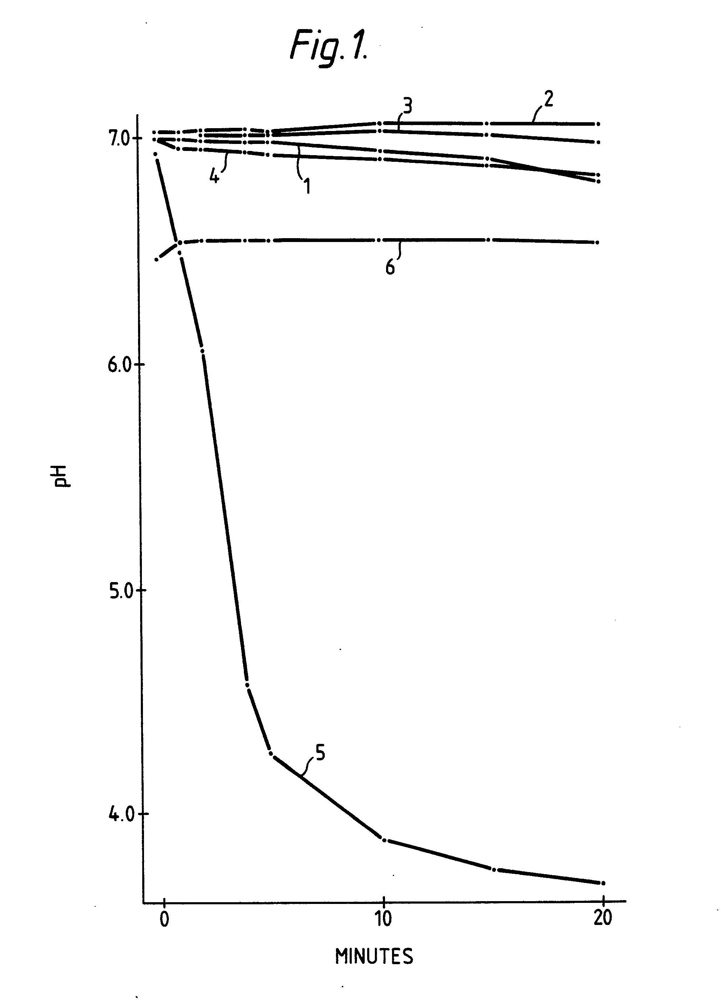

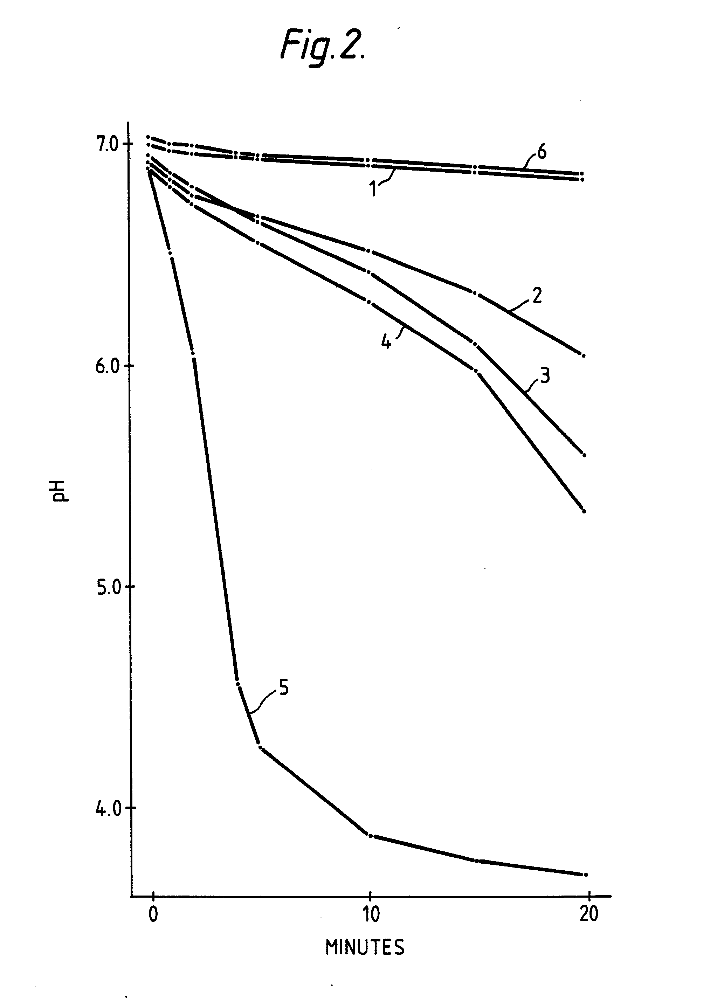

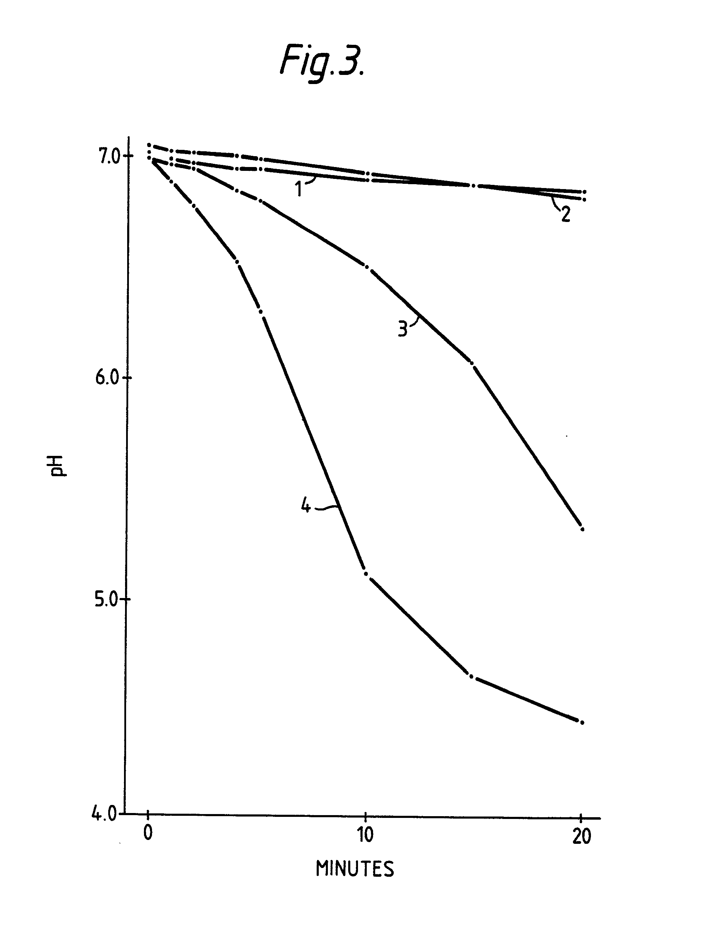

CLAIMS (for states other than Austria)

1. A composition for reducing the incidence or severity of human dental caries, the composition comprising an aminated cariogen or a pharmaceutically acceptable salt thereof in association with a pharmaceutically acceptable carrier.

2. A composition as claimed in claim 1, wherein the aminated cariogen is an aminated derivative of a monosaccharide.

3. A composition as claimed in claim 2, wherein the aminated cariogen is glucosamine, N-acetylglucosamine or mannosamine.

4. For use in reducing the incidence or severity of human dental caries, an aminated cariogen or a pharmaceutically acceptable salt thereof.

5. For use in reducing the incidence or severity of human dental caries, a composition comprising an aminated cariogen or a pharmaceutically acceptable salt thereof in association with a pharmaceutically acceptable carrier.

0057323

CLAIMS (for Austria)

1.    A method of preparing a composition for reducing the incidence or severity of human dental caries, the method comprising associating an aminated cariogen or a pharmaceutically acceptable salt thereof with a pharmaceutically acceptable carrier.

2. A method as claimed in claim 1, wherein the aminated cariogen is an aminated derivative of a monosaccharide.

3. A method as claimed in claim 2, wherein the aminated cariogen is glucosamine, N-acetylglucosamine or mannosamine.

Fig.1.

Fig.2.

Fig.3.